# EUROPEAN PATENT APPLICATION

(11) **EP 1 752 101 A2**
(43) Date of publication of application: **14.02.2007**
(21) Application number: 06015538.9
(22) Date of filing: 26.07.2006
(51) Int. Cl.: A61B 8/00, G01S 15/89

(54) **Control panel for use in an ultrasonic diagnostic apparatus**

(30) Priority: 08.08.2005 KR 20050022870; 09.09.2005 KR 20050084087
(71) Applicant: MEDISON CO., LTD., Kangwon-do 250-870 (KR)
(72) Inventor: Lee, Hong Gyo Discusser&Medison Building, Gangnam-gu, Seoul 135-280 (KR); Lee, Dong Hyun Discusser&Medison Building, Gangnam-gu, Seoul 135-280 (KR)
(74) Representative: Lorenz, Markus

(57) **Abstract**

The present invention relates to a control panel for use in an ultrasonic diagnostic apparatus, which enables a user to input various commands via one input device, thereby providing convenience in use. The ultrasonic diagnostic apparatus includes a control part for controlling operations thereof and the control panel is configured to transfer an input made by a user to the control part. The control panel comprises: a first input device having a pointing function or a scrolling function; and a second input device disposed around the first input device, the second input device having a plurality of input sections and generating a signal when the user manipulates each input section. A command corresponding to the signal from the second input device is key-mapped by the control part.

## Description

The present invention generally relates to a control panel for use in an ultrasonic diagnostic apparatus, and more particularly to a control panel for use in an ultrasonic diagnostic apparatus that enables a user to input various commands by using one input device.

An ultrasonic diagnostic apparatus is a medical device for obtaining an ultrasound image of a target region in an object. By obtaining such an image, clinical information of the target region (e.g., lesion or neoplasm information of internal organs, fetus information, etc.) can be provided.

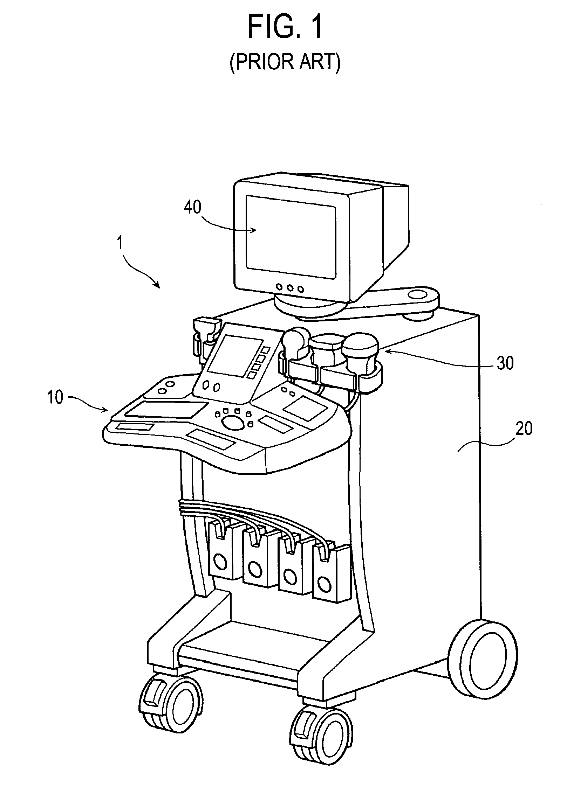

Fig. 1 illustrates a conventional ultrasonic diagnostic apparatus 1, which comprises: a main body 20; a probe 30 for radiating an ultrasonic wave to a target region and receiving an echo signal reflected from the target region; a monitor 40 for displaying ultrasound images; and a control panel 10 for inputting commands.

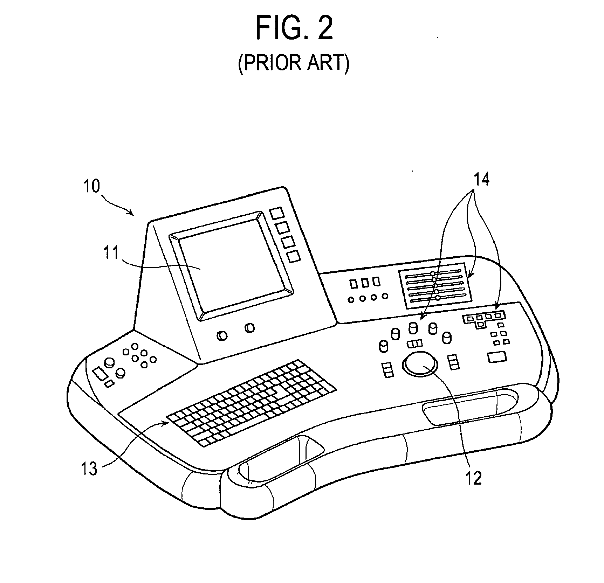

Fig. 2 illustrates the control panel of the conventional ultrasonic diagnostic apparatus. The control panel 10 includes a touchscreen 11, a trackball 12, a keyboard 13, a plurality of keys/terminals 14 and the like. For example, the touchscreen 11 displays the operating state of the conventional ultrasonic diagnostic apparatus 1 and generates a signal by a user's touch. The trackball 12 may be used for pointing or scrolling purposes. The keyboard 13 may be used for inputting letters or figures. The keys/terminals 14 may be used for selecting a diagnosis mode or adjusting output signals or ultrasound images.

When the user performs an ultrasonic diagnosis, the user generally holds the probe in one hand and moves the probe upon the skin of a patient. The user then generally manipulates the input devices (i.e., the touchscreen 11, the trackball 12, the keyboard 13 or the keys/terminals 14) of the control panel 10 with the other hand.

However, since the input devices are scattered on the control panel, it is inconvenient and troublesome to manipulate the different types of input devices. Thus, performing the ultrasonic diagnosis can take a very long time.

The present invention provides a control panel of an ultrasonic diagnostic apparatus that enables a user to input various commands through one input device without needing to handle several input devices at once, thereby providing convenience in use.

In accordance with the invention as embodied broadly herein, there is provided a control panel for use in an ultrasonic diagnostic apparatus. The ultrasonic diagnostic apparatus includes a control part for controlling the operations thereof and the control panel is configured to transfer an input made by a user to the control part. The control panel comprises the following: a first input device having a pointing function or a scrolling function; and a second input device disposed around the first input device and having a plurality of input sections, the second input device being configured to generate a signal when the user manipulates each input section, wherein a command corresponding to the signal from the second input device is key-mapped by the control part.

In the present invention, the second input device includes: an annular pad disposed around the first input device; and at least one sensor disposed under the annular pad for generating the signal according to a pressure exerted to the annular pad and transferring the signal to the control part.

The second input device may further include: an annular dial disposed around the first input device; and a plurality of switches disposed under the annular dial for generating the signal when the annular dial contacts thereto and transferring the signal to the control part.

The control panel further comprises a device for indicating the command, which is key-mapped by the control part. The command-indicating device is controlled by the control part.

The command-indicating device includes: a plurality of LEDs positioned adjacent to the respective input sections; and a plurality of labels for representing the key-mapped command. Each label is provided on the control panel corresponding to each LED.

The command-indicating device includes a display device selected from the group consisting of a 7-segment display device, a dot matrix display device and a liquid crystal display device.

When the command, which corresponds to the signal from the second input device, is a diagnosis mode, the control part matches a subsequent signal from the second input device to a subcommand subordinate to the chosen diagnosis mode.

The above features of the present invention will become more apparent from the following descriptions of the preferred embodiments given in conjunction with the accompanying drawings.
Fig. 1 is a perspective view illustrating a conventional ultrasonic diagnostic apparatus.
Fig. 2 is a perspective view illustrating a conventional control panel of the ultrasonic diagnostic apparatus shown in Fig. 1.
Fig. 3 is a partial perspective view illustrating a control panel constructed in accordance with a first preferred embodiment of the present invention.
Fig. 4 is a schematic cross-sectional view along the line A-A in Fig. 3.
Fig. 5 is a plan view of the control panel shown in Fig. 3.
Fig. 6 is a block diagram illustrating the constitution of the control panel shown in Fig. 3.
Fig. 7 is a partial plan view of the control panel showing an alternative configuration of a second input device.
Fig. 8 is a partial perspective view illustrating a control panel constructed in accordance with a second preferred embodiment of the present invention.
Fig. 9 is a schematic cross-sectional view along the line B-B in Fig. 8.
Figs. 10 and 11 are partial plan views of a control panel constructed in accordance with a third preferred embodiment of the present invention.
Fig. 12 is a block diagram illustrating the constitution of the control panel shown in Figs. 10 and 11.

The preferred embodiments of the present invention will now be described with reference to the accompanying drawings.

A control panel of the present invention is employed in an ultrasonic diagnostic apparatus. The ultrasonic diagnostic apparatus has a control part (not shown) for controlling the operations related to ultrasonic diagnosis. Alternatively, the control part may be provided in the control panel. The control panel of the present invention may have various input devices thereon, each of which can transfer an input made by the user to the control part as a signal. The input device may include a touchscreen, a trackball, a keyboard, keys, terminals and the like.

The control panel of the present invention further includes another input device, which is configured to have a plurality of input sections as well as to sense the input made by the user at each input section, thereby enabling a multiple input. Each signal from each input section can be matched to a specific command by the control part. Such function of the control part, wherein a specific command is performed in response to a specific signal by the control part, is generally known as "key-mapping."

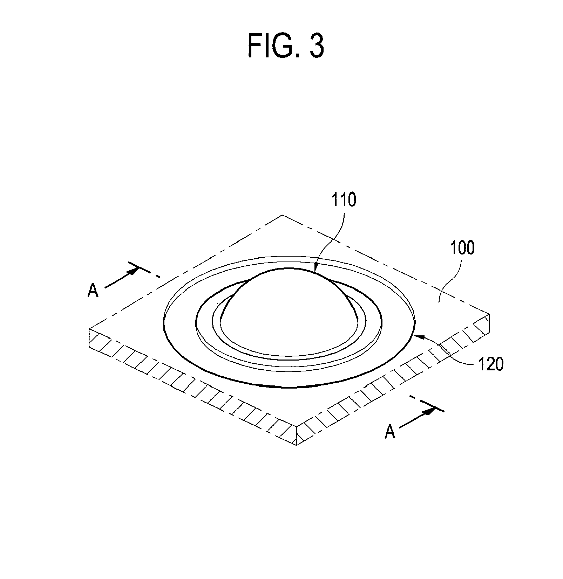

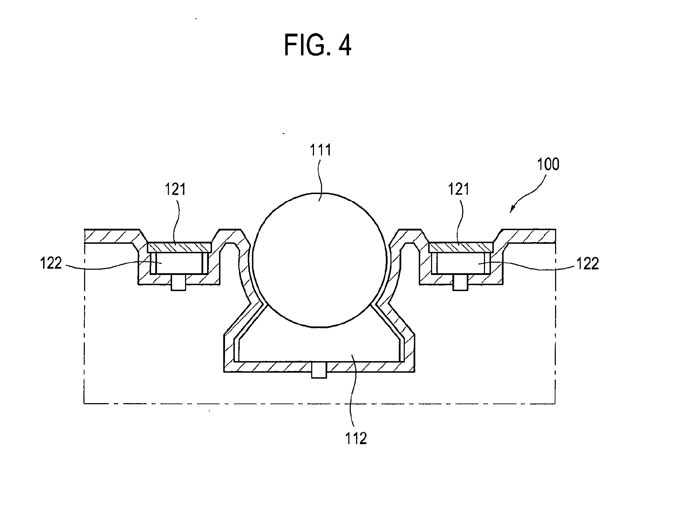

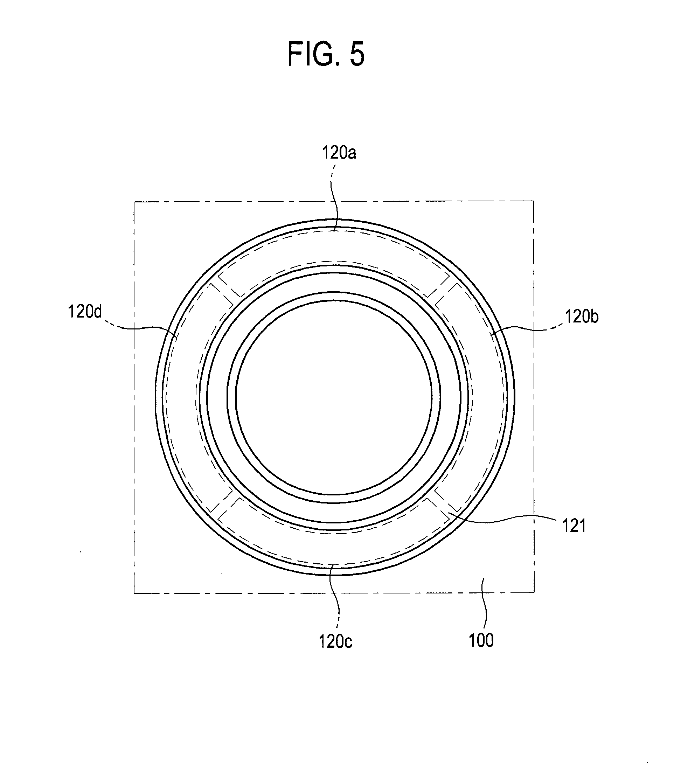

Fig. 3 shows a partial perspective view of a control panel constructed in accordance with a first preferred embodiment of the present invention. Fig. 3 shows a portion of the control panel with another input device provided thereon. Fig. 4 shows a schematic cross-sectional view along the line A-A in Fig. 3. Fig. 5 shows a plan view of the control panel shown in Fig. 3.

Referring now to Figs. 3 to 5, the control panel 100 of the present invention comprises: a first input device 110 having a pointing function and/or a scrolling function; and a second input device 120 disposed around the first input device 110 and enabling a multiple input.

In the present invention, the first input device 110 is preferably a trackball input device. The first input device 110 has pointing and scrolling functions. The first input device 110 includes a ball 111 to be rolled by the user and a sensing part 112 disposed under the ball 111 for sensing the movement of the ball 111. The sensing part 112 senses the movement of the ball 111 caused by the user and transfers a corresponding signal to the control part. The first input device 110 serves to move a pointer displayed on the touchscreen or scroll diagnostic records of a patient to be displayed on the touchscreen.

The second input device 120 can sense the user's touch or rubbing similar to a "touchpad." The second input device 120 includes: an annular pad 121 that can be touched or rubbed with the user's finger; and a sensing part 122 disposed under the annular pad 121 and generating a signal. The sensing part 122 is configured to sense the changes in pressure exerted onto the annular pad 121 when the user touches or rubs the annular pad 121 and to generate a signal corresponding thereto. As for the sensing part 122, a plurality of sensors for sensing pressure may be disposed along the circumferential direction of the annular pad 121. Alternatively, a single sensor, which has an annular shape similar to the annular pad 121 and is configured to sense pressure, may be disposed.

Since the second input device 120 is configured to have an annular shape, the second input device 120 can be divided into several input sections. The signals generated from the respective input sections of the second input device 120 are key-mapped by the control part and different commands corresponding to the signals are performed. Accordingly, the second input device 120 enables a multiple input. The second input device 120 will now be described in detail by way of an example in which it is divided into four input sections.

As shown in Fig. 5, the second input device 120 includes four input sections 120a to 120d, through which at least four different inputs can be made. Also, four different commands can be performed by means of key-mapping of the control part. Accordingly, the user is relieved from the inconvenience of handling several input devices scattered on the control panel when operating the ultrasonic diagnostic apparatus.

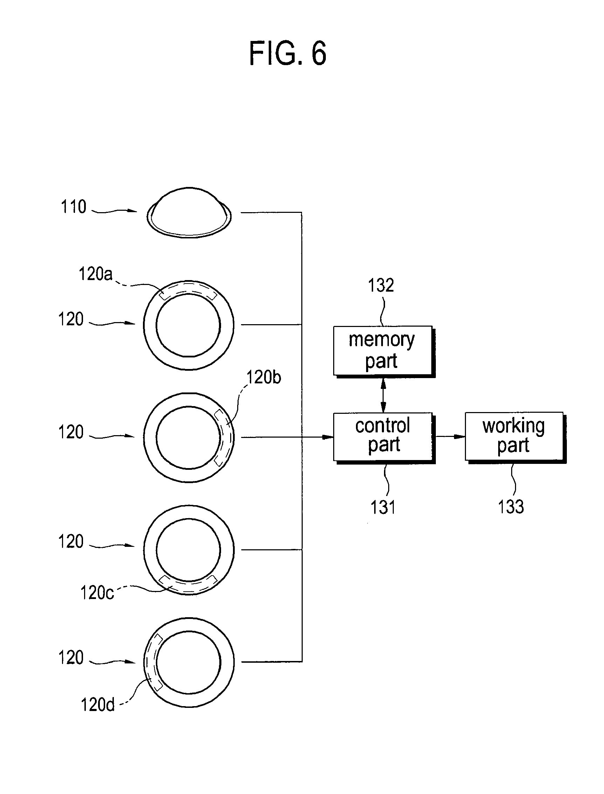

Fig. 6 is a block diagram that illustrates the constitution of the control panel shown in Fig. 3. When the user touches or rubs the surface of the annular pad 121 relating to the first input section 120a of the second input device 120, the sensing part 122, which is positioned under the annular pad 121 relating to the first input section 121, senses an input made by the user. The sensing part 122 transfers a signal, which corresponds to such input, to the control part 131. The control part 131 selects a command corresponding to the signal from the first input section 120a after interacting with a memory part 132 and then outputs the selected command to a working part 133. As a result, the ultrasound diagnostic apparatus performs an operation that is associated with the selected command. The control part 131 is preset or programmed so as to match the signal from each input section to the command related thereto as well as to perform the selected command. The commands corresponding to the signals from the second input device 120 are saved in the memory part 132. Such commands may be performed when the user handles various input devices (i.e., touchscreen, keyboard, keys, terminals, etc), which may be provided on the control panel 100.

In actual ultrasonic diagnosis, the signal from any input section of the second input device 120 can be key-mapped so as to correspond to a command for choosing a diagnosis mode. In such a case, if the user chooses any one of the diagnosis modes, then the control part 131 can match each subsequent signal from each input section 120a to 120d to each subcommand subordinate to the chosen diagnosis mode. For example, in case any one of the diagnosis modes is chosen, the signal from the first input section 120a can be key-mapped so that the depth of an ultrasonic image can be changed. Also, the signal from the second input section 120b can be key-mapped such that the zoom effect such as "zoom in" or "zoom out" can be provided. Further, the signal from the third or fourth input section 120c or 120d can be key-mapped such that the chosen mode may be changed into any other diagnosis mode. In the case of the depth change or zoom effect, these operations can be made when the user rubs the first or second input section 120a or 120b with his/her finger. Particularly, when the user touches the second input section 120b, all the input sections of the second input device 120 may be key-mapped once again such that "zoom in" or "zoom out" can be performed according to the rubbing direction.

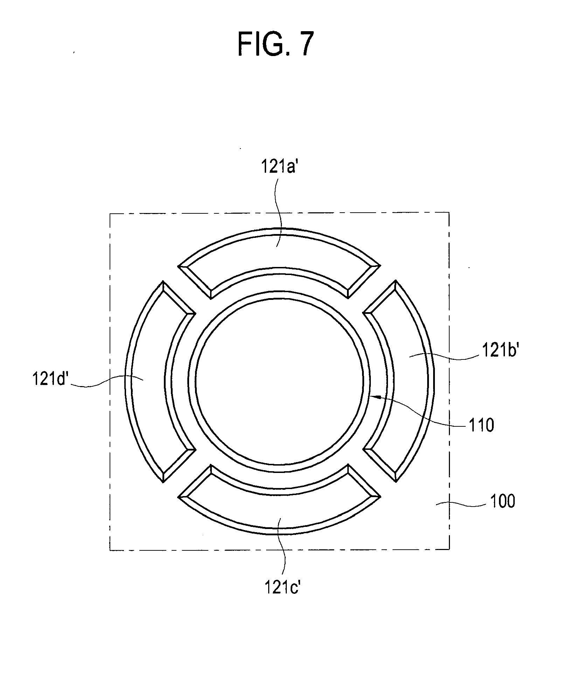

Fig. 7 is a partial plan view of the control panel 100 showing an alternative configuration of the second input device 120. A number of sectioned pads may be arranged around the first input device 110. Referring now to Fig. 7, four sectioned pads 121a' to 121d' are provided around the first input device 110 on the control panel 100. Since four sectioned pads 121 a' to 121 d' are separately arranged around the first input device 110, the user can handle each sectioned pad 121a' to 121d' with more definite touching sensations.

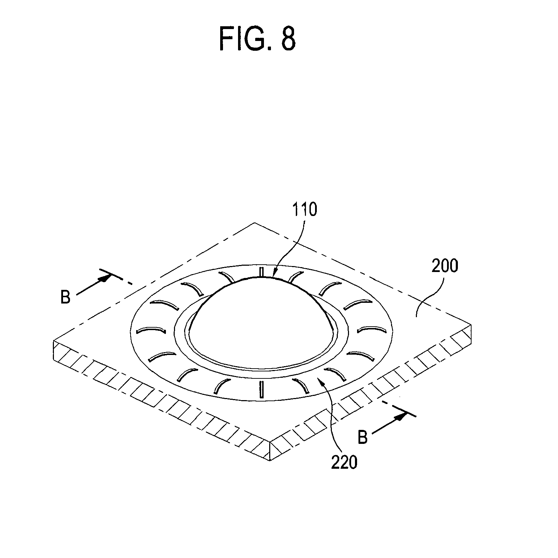

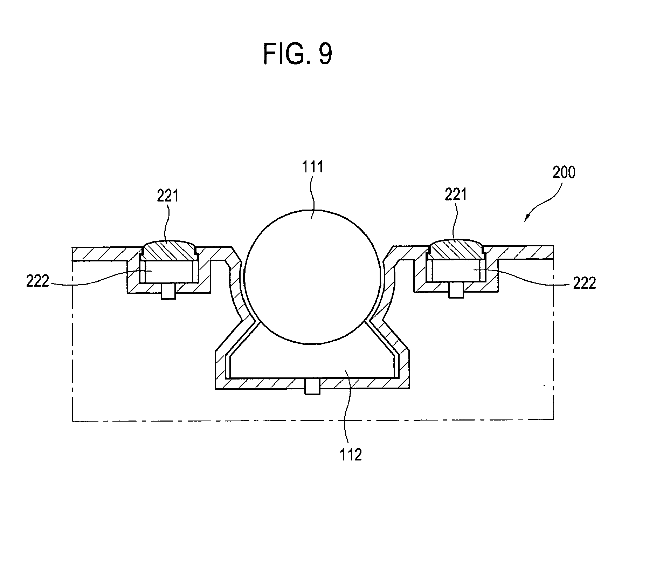

Fig. 8 is a partial perspective view illustrating a control panel, which is in accordance with a second preferred embodiment of the present invention. Fig. 9 is a schematic cross-sectional view along the line B-B in Fig. 8.

In addition to the first input device 110, the control panel 200 comprises a second input device 220 capable of sensing presses and turns caused by the user (similar to a "jog dial"). The second input device 220 includes a dial 221 having an annular disk shape and a sensing part 222 disposed along and under the dial 221. Accordingly, in the present embodiment, the user makes inputs by pressing a portion of the dial 221 or turning the dial 221 in one direction.

The sensing part 222 comprises a plurality of switches, which can be activated to generate a signal when a portion of the dial 221 is pressed down by the user and contacts one or more switches.

Since the second input device 220 is configured to have an annular shape, the second input device 220 can be divided into several input sections. When the user presses down any portion of the dial 221 relating to any one of the input sections, the switch/switches 222 relating to the input section becomes activated to generate a signal. A command associated with such input section can then be performed through the key-mapping of the control part. Accordingly, the second input device 220 enables a multiple input. Also, when the second input device 220 is turned in one direction by the user, a signal related to the turning direction of the second input device 220 is generated, while some switches 222 are sequentially activated along the turning direction. Therefore, a command such as "zoom in", "zoom out" or depth change can be performed.

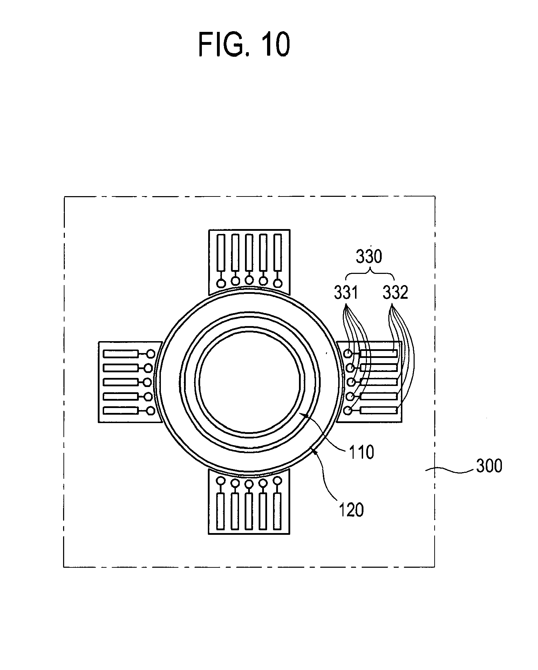

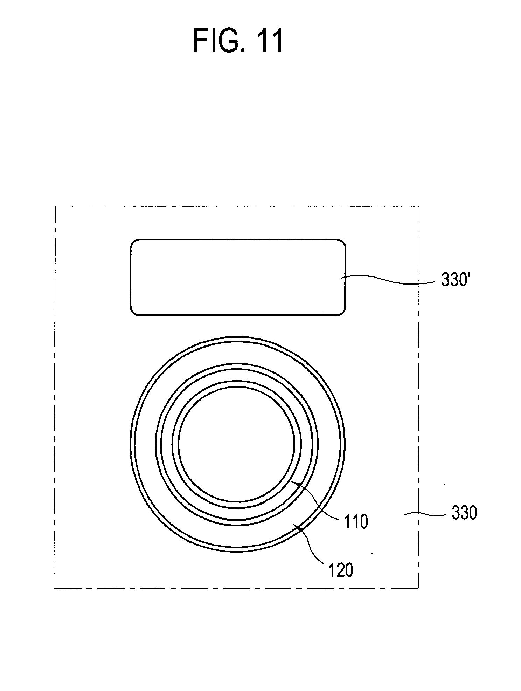

Figs. 10 and 11 are partial plan views of a control panel, which is in accordance with a third preferred embodiment of the present invention. The control panel 300 of the present embodiment comprises: the first input device 110; the second input device 120 disposed around the first input device 110 (as described above); and a command-indicating device 330 or 330' disposed adjacent to the second input device 120 for indicating key-mapped commands when the user handles the second input device 120.

The control panel 300 shown in Fig. 10 is provided with the command-indicating devices 330, which are arranged adjacent to each input section of the second input device 120. The command-indicating device 330 includes a plurality of LEDs 331 and a plurality of labels 332 attached to the control panel 300 so as to correspond to each LED. On each label 332, words or symbols are printed to indicate a command, which can be performed through key-mapping when the user touches one of the input sections of the second input device 120. If the user handles the second input device 120, then the command associated with the input section of the second input device 120 is performed through key-mapping. At the same time, the LED, which corresponds to the label 332 having the words or symbols indicating such command, is turned on. Then, the user reads the words or symbols of the label adjacent to the LED. By doing so, it becomes possible to indicate the selected command.

The control panel 300 shown in Fig. 11 is provided with a command-indicating device 330' such as a display device. The command-indicating device 330' may be disposed adjacent to the second input device 120 or in any suitable place on the control panel 300 where the user can readily see it. The command-indicating device 330' includes a 7-segment display device, a dot matrix display device or a liquid crystal display device. Accordingly, the command-indicating device 330' can display the command, which is selected when the user handles the second input device 120, as words or symbols. Particularly, when a liquid crystal display device is employed, since each selected command can be displayed as a symbol or an icon, the user can recognize the selected command more visually.

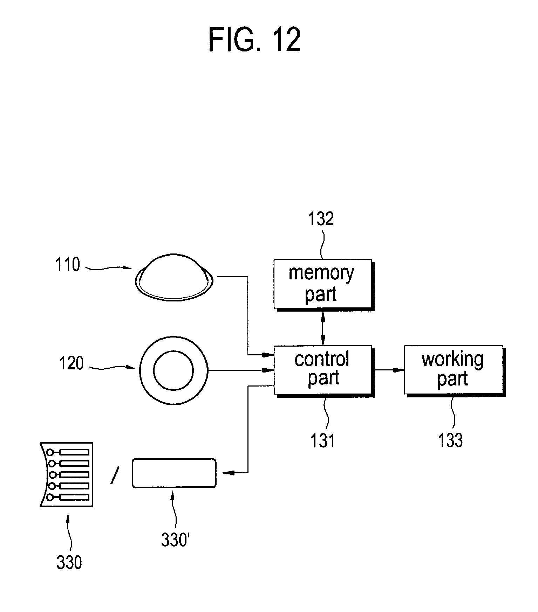

Fig. 12 is a block diagram showing the constitution of the control panel 300, which is shown in Figs. 10 and 11. The command-indicating device 330 or 330' is controlled by the control part 131. When the signal is generated from any input section of the second input device 120, the control part 131 activates the command-indicating device 330 or 330' to thereby indicate the command selected through key-mapping. Alternatively, in case the control panel 300 is provided with the touchscreen 11 (shown in Fig. 2), the control part 131 can enable the touchscreen 11 to directly display the selected command thereon without necessitating the command-indicating device 330 or 330'.

As described above in detail, the control panel, which is in accordance with the present invention, enables a user to input various commands via one input device without needing to handle several input devices at once. Consequently, convenience in use is enhanced and rapid ultrasonic diagnosis can be provided.

While the present invention has been described and illustrated with respect to a preferred embodiment of the invention, it will be apparent to those skilled in the art that variations and modifications are possible without deviating from the broad principles and teachings of the present invention, which should be limited solely by the scope of the claims appended hereto.

## Claims

1. A control panel for use in an ultrasonic diagnostic apparatus, the ultrasonic diagnostic apparatus including a control part for controlling operations thereof and the control panel being configured to transfer an input made by a user to the control part,
the control panel comprising:
a first input device having a pointing function or a scrolling function; and
a second input device disposed around the first input device, the second input device having a plurality of input sections and generating a signal when the user manipulates each input section;
wherein a command corresponding to the signal from the second input device is key-mapped by the control part.

2. The control panel of Claim 1, wherein the second input device includes: an annular pad disposed around the first input device; and at least one sensor disposed under the annular pad for generating the signal according to a pressure exerted to the annular pad and transferring the signal to the control part.

3. The control panel of Claim 1, wherein the second input device includes: an annular dial disposed around the first input device; and a plurality of switches disposed under the annular dial for generating the signal when the annular dial contacts thereto and transferring the signal to the control part.

4. The control panel of Claim 1, wherein the control panel further comprises a device for indicating the command key-mapped by the control part, the command-indicating device being controlled by the control part.

5. The control panel of Claim 4, wherein the command-indicating device includes: a plurality of LEDs disposed adjacent to the respective input sections; and a plurality of labels for representing the key-mapped command, wherein each label is attached to the control panel corresponding to each LED.

6. The control panel of Claim 4, wherein the command-indicating device includes a display device selected from the group consisting of a 7-segment display device, a dot matrix display device and a liquid crystal display device.

7. The control panel of Claim 1, wherein when the command corresponding to the signal from the second input device is a diagnosis mode, the control part matches a subsequent signal from the second input device to a subcommand subordinate to the chosen diagnosis mode.
